(19) Europäisches Patentamt
European Patent Office
Office européen des brevets

(11) EP 4 091 676 A1

(12) EUROPÄISCHE PATENTANMELDUNG

(43) Veröffentlichungstag:
**23.11.2022 Patentblatt 2022/47**

(21) Anmeldenummer: **22167866.7**

(22) Anmeldetag: **12.04.2022**

(51) Internationale Patentklassifikation (IPC):
***A61Q 5/12*** *(2006.01)* ***A61K 8/34*** *(2006.01)*
***A61K 8/37*** *(2006.01)* ***A61K 8/42*** *(2006.01)*

(52) Gemeinsame Patentklassifikation (CPC):
**A61Q 5/12; A61K 8/342; A61K 8/37; A61K 8/42;**
A61K 2800/34; A61K 2800/5922

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **12.05.2021 DE 102021204836**

(71) Anmelder: **Beiersdorf AG**
**20253 Hamburg (DE)**

(72) Erfinder:
- **Saß, Viola**
**25436 Tornesch (DE)**
- **Reiter, Katharina**
**21357 Barum (DE)**

(74) Vertreter: **Beiersdorf AG**
**Patentabteilung**
**Unnastraße 48**
**20245 Hamburg (DE)**

(54) **HAAR-KOSMETISCHE ZUBEREITUNG ZUR KONDITIONIERUNG DER HAARE II**

(57) Conditioner-Zubereitung für die Haare, die Silikon-frei sind, gleichwohl aber eine sehr gute Pflegeleistung aufweisen und eine angenehme Textur haben.

EP 4 091 676 A1

## Beschreibung

Die vorliegende Erfindung beschreibt Conditioner für die Haare, die Silikon-frei sind, gleichwohl aber eine sehr gute Pflegeleistung aufweisen und eine angenehme Textur haben.

Conditioner, auch als Haarspülungen bezeichnet, werden in der Regel nach der Reinigung der Haare angewendet. Conditioner sind an sich bekannt und sollen das Haar nach der Haarwäsche pflegen. Aus der großen Zahl an Dokumenten des Standes der Technik seien hier nur wenige als Beispiel genannt.

Das Dokument EP 2138156 A1 offenbart Haar-konditionierende Mittel, die verschiedene kationische Tenside in einem bestimmten Verhältnis enthalten.

Im Dokument EP 0786250 A1 werden u.a. Haarspülungen beschrieben, die ein Alkylamidoamin, Esterquats und Fettalkohole enthalten.

Das Dokument DE 102017212403 A1 offenbart Haarpflegemittel, die neben ausgewählten Alkanen Amidoamine enthalten.

Durch eine Haarwäsche wird das Haar gereinigt, d.h. es werden Schmutzpartikel und/oder -filme, die von außen auf das Haar gelangt sind, entfernt. Gleichzeitig wird aber auch die äußere Schicht des Haares, die Cuticula, angegriffen. Die Cuticula weist auf der Oberfläche kovalent gebundene Fettsäuren auf, die zur Hydrophobizität der Cuticula beitragen. Durch den Waschvorgang können zumindest zu einem gewissen Teil diese Fettsäuren ausgewaschen werden. Dieser Prozess kann bewirken, dass das Haar trocken wird.

Um diesem Phänomen entgegenzuwirken, verwenden viele Verbraucher/innen einen Conditioner nach der Haarwäsche. Der Zweck der Verwendung dieser Haar-kosmetischen Zubereitungen ist es, dem Haar idealerweise die Komponenten zurückzugeben, die möglicherweise durch die Haarwäsche mit ausgewaschen wurden. Deshalb sind im allgemeinen lipophile Komponenten in Conditionern enthalten, die mit den lipophilen Verbindungen der Cuticula wechselwirken können.

Neben der Hydrophobizität der Cuticula, ist auch die Proteinstruktur der Cuticula zu beachten. Die Zusammensetzung der Proteinstruktur resultiert in einem isoelektrischen Punkt von etwa 3,67 an der Haaroberfläche. Der pH-Wert, den die üblicherweise eingesetzten Haar-kosmetische Zubereitungen aufweisen, bewirkt, dass negativ geladene Strukturen auf der Haaroberfläche vorliegen.

Pflegende Komponenten in Conditioner-Zubereitungen umfassen also lipophile Verbindungen, die mit den lipophilen Verbindungen an der Cuticula-Oberfläche in Wechselwirkung treten und/oder positive geladene Verbindungen (kationische Verbindungen), die mit den negative geladenen Strukturen auf der Haaroberfläche wechselwirken.

In der Vergangenheit wurden vielfach Silikon-basierte Verbindungen als Pflegekomponenten in Conditioner-Zubereitungen eingesetzt. Silikone können als Silikonöle vorliegen, die hydrophob sind und mit den lipophilen Komponenten auf der Haaroberfläche wechselwirken und idealerweise einen Schutzfilm auf der Haaroberfläche ausbilden.

Silikonverbindungen können substituiert sein und zwar mit chemischen Gruppen, die positive Ladungen aufweisen (kationische Silikonverbindungen). Diese Silikonverbindungen können auch mit den negativ geladenen Strukturen an der Haaroberfläche in Wechselwirkung treten.

Neben Silikonverbindungen werden vielfach auch kationische Polymere als pflegende Komponenten eingearbeitet. Hierbei handelt es sich in der Regel um Polymere, die zumeist synthetischen Ursprungs sind. Diese kationische Pflegepolymere können ebenfalls mit den negativ geladenen Oberflächenstrukturen des Haares wechselwirken.

Die Conditioner-Zubereitungen der Vergangenheit hatten somit den Focus, die Haare zu pflegen. Der Einsatz der oben genannten großen Klassen von Pflegekomponenten kann jedoch auch Nachteile haben. Teilweise leidet die Textur dieser Zubereitungen; sie lassen sich dann nur schwer aus dem Verpackungsmittel entnehmen und schlecht im Haar verteilen. Zudem beschreiben die Verbraucher derartige Zubereitungen als "schwer", d.h. die Zubereitungen haben eine fast pastöse Textur. Ein weiterer Nachteil, der oft bei Silikon-haltigen Zubereitungen beobachtet wird, ist es, dass die Haare "beschwert" sind, d.h. der Film auf den Haaren wurde bei der Haarwäsche nicht vollständig weggewaschen und baute sich immer weiter auf. Eine Frisurgestaltung ist dann überaus schwierig.

Als weiterer Aspekt ist auch das zunehmende Umweltbewusstsein zu nennen. Der Verbraucher wünscht sich Zubereitungen, die natürliche Inhaltsstoffe enthalten und die biologische Abbaubarkeit soll auch gegeben sein. Silikonverbindungen und viele kationische Polymere sind jedoch synthetischer Natur und weisen teilweise eine schlechte biologische Abbaubarkeit auf.

Um die oben genannten Nachteile zu überwinden und zunehmend umwelt-freundlichere Conditioner-Zubereitungen zur Verfügung zu stellen, die eine leichte Textur aufweisen und frei von Silikonverbindungen, synthetischen kationischen Pflegepolymeren und alkoxylierten Verbindungen sein sollten, wurden neue Conditioner-Zubereitungen entwickelt und beschrieben, siehe dazu die PCT-Anmeldungen mit den Anmeldenummern PCT/CN2019/121114 und PCT/CN2019/121115. Beide Zubereitungen enthalten eine spezielle Mischung von Fettalkoholen, bestimmte Emollienzien und ausgewählte Emulgatoren.

Produkte gemäß der genannten Erfindung wurden als leicht empfunden, beziehungsweise ließen sich schnell ausspülen.

Für Verbraucher, speziell mit feinen und/oder strapazierten oder geschädigten Haaren waren diese Produkte jedoch nicht befriedigend in Bezug auf pflegenden Eigenschaften.

Die Aufgabe der vorliegenden Erfindung bestand nun darin, Conditioner-Zubereitungen für die menschlichen Haare zur Verfügung zu stellen, die zum einen Silikon-frei sein, eine ansprechende Textur haben und weitgehend umweltfreundlich sein sollten und zum anderen eine hinreichende Pflegeleistung erbringen sollten. Es galt nun beide, an sich gegenläufigen, Ziele in einer Zubereitung zu verwirklichen.

Überraschenderweise konnte dies durch eine kosmetische Conditioner-Zubereitung für die menschlichen Haaren, insbesondere feine und/oder strapazierte oder geschädigte Haare, enthaltend

- eine Kombination von Fettalkoholen, wobei ein oder mehrere Fettalkohol(e) aufweisend ≥ 18 Kohlenstoffatome mit einem Gesamtgehalt von weniger als 1,5 Gew.-% und ein oder mehrere Fettalkohol(e) aufweisend ≤ 16 Kohlenstoffatome mit einem Gesamtgehalt von weniger als 8,0 Gew.-% vorliegen, jeweils bezogen auf das Gesamtgewicht der Zubereitung,

- wenigstens ein Esteröl, wobei das wenigstens eine Esteröl Ester von einwertigen Alkoholen mit Fettsäuren umfasst und

- wenigstens ein Alkylamidoamin,

verwirklicht werden.

Die erfindungsgemäße Zubereitung ist eine wässrige Zubereitung, vorteilhaft beträgt der Wassergehalt von 60 bis 95 Gew.-%, insbesondere von 80 bis 90 Gew.-%, bezogen das Gesamtgewicht der Zubereitung.

Die erfindungsgemäße Zubereitung liegt vorteilhaft in Form einer Emulsion vor, insbesondere in Form einer O/W-Emulsion.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z.B. Butter) handelt es sich um das umgekehrte Prinzip, wobei der Grundcharakter hier durch das Öl bestimmt wird.

Emulsionen benötigen zu ihrer Bildung und zur Stabilisierung im Allgemeinen einen oder mehrere Emulgatoren, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe). Dabei kann zwischen nicht-ionischen, anionischen und kationischen Emulgatoren unterschieden werden.

Ein Kennzeichen für die Hydrophilie eines gegebenen Emulgators ist dessen HLB-Wert, der mithilfe nachfolgender Formel bestimmt werden kann:

$$HLB = 20 \times (1 - M_{lipophil}/M),$$

wobei $M_{lipophil}$ für die Molmasse des lipophilen Anteils im Emulgator und
M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis etwa 15 auf. Substanzen mit HLB-Werten größer 15 werden häufig als Lösungsvermittler bezeichnet.

Es ist vorteilhaft hydrophile Emulgatoren ausgewählt, also solche, die einen HLB-Wert von ≥ 10 haben. Weiter vorteilhaft wird der Emulgator Polyglyceryl-3 Methylglucose Distearat gewählt. Der genannte Emulgator kann beispielsweise unter der Handelsbezeichnung Tego Care 450 bei der Firma Evonik (Polyglyceryl-3 Methylglucose Distearate) bezogen werden.

Wenn in der erfindungsgemäßen Conditioner-Zubereitung wenigstens ein Emulgator enthalten ist, so liegt der wenigstens eine Emulgator mit einem Gesamtgehalt von 0,05 bis 1,0 Gew.-%, bevorzugt 0,1 bis 0,4 Gew.-% vor, bezogen auf das Gesamtgewicht der Conditioner-Zubereitung.

Die erfindungsmäße Conditioner-Zubereitung enthält eine Kombination von Esterölen. Esteröle können den Emollientien zugerechnet werden. Unter Emollientien werden Substanzen verstanden, die das Haart weich und geschmeidig machen. Es handelt sich dabei vorteilhaft um lipophile Komponenten.

Im allgemeinen sind Esteröle Ester von gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen ausgewählt werden. Trotz des Begriffs Esteröl können diese Substanzen flüssig oder fest sein.

Erfindungsgemäß enthält die Conditioner-Zubereitung wenigstens ein Esteröl in Form von Estern gebildet aus einem einwertigen Alkohol mit einer Fettsäure.

Die Alkohole, die zur Ausbildung der Ester verwendet werden, sind einwertige Alkohole mit 2 bis 24 Kohlenstoffatomen, bevorzugt mit 2 bis 10 Kohlenstoffatomen. Besonders bevorzugt ist es, wenn die einwertigen Alkohole aus verzweigten Alkoholen mit 2 bis 10 Kohlenstoffatomen ausgewählt werden.

Die Fettsäuren, die zur Ausbildung der Ester verwendet werden, sind Fettsäuren mit 6 bis 30 Kohlenstoffatomen, bevorzugt mit 6 bis 18 Kohlenstoffatomen. Besonders bevorzugt ist es, wenn unverzweigte und gesättigte Fettsäuren mit 6 bis 18 Kohlenstoffatomen gewählt werden.

Eine insbesondere bevorzugte Gruppe der der Esteröle umfasst, weiter insbesondere besteht aus, Isopropylmyristat, Isopropylpalmitat, Isopropylstearat, 2-Ethylhexylpalmitat, 2-Ethylhexyllaurat, 2-Ethylhexylstearat, und/oder Isoamyllaurat. Am meisten bevorzugt sind Isoamyllaurat, Isopropylmyristat und/oder Isopropylpalmitat.

Isoamylalkohol ist beispielsweise unter dem Handelsnamen Mackaderm bei der Firma Solvay erhältlich.

In der erfindungsgemäßen Conditioner-Zubereitung ist das wenigstens ein Esteröl in Form von Estern gebildet aus einem einwertigen Alkohol mit einer mit einem Gesamtgehalt von 0,1 bis 2,0 Gew.-%, bevorzugt 0,5 bis 1,0 Gew.-% enthalten, bezogen auf das Gesamtgewicht der Zubereitung.

In der erfindungsgemäßen Conditioner-Zubereitung ist eine Kombination von Fettalkoholen enthalten, wobei ein oder mehrere Fettalkohol(e) aufweisend ≥ 18 Kohlenstoffatome mit einem Gesamtgehalt von weniger als 1,5 Gew.-% und ein oder mehrere Fettalkohol(e) aufweisend ≤ 16 Kohlenstoffatome mit einem Gesamtgehalt von weniger als 8,0 Gew.-% enthalten sind, jeweils bezogen auf das Gesamtgewicht der Zubereitung.

Fettalkohole sind aliphatische, langkettige, einwertige, meist primäre Alkohole. Der Begriff Fettalkohol wird in der Regel für Fettalkohole mit einer Kettenlänge von 6 bis 22 Kohlenstoffatomen verwendet. Die Kohlenstoffkette der Fettalkohole kann eine oder mehr Doppelbindungen aufweisen und/oder unverzweigt oder verzweigt sein.

Es ist bevorzugt, wenn in der erfindungsgemäßen Conditioner-Zubereitung der oder die Fettalkohol(e) unverzweigt und/oder gesättigt ist/sind, weiter bevorzugt unverzweigt und gesättigt.

Der oder die Fettalkohol(e) aufweisend ≥ 18 Kohlenstoffatome wird/werden weiter bevorzugt aus der Gruppe Stearylalkohol, Arachidylalhohol und/oder Behenylalkohol ausgewählt, besonders bevorzugt ist Stearylalkohol.

Der oder die Fettalkohol(e) aufweisend ≤ 16 Kohlenstoffatome wird/werden weiter bevorzugt aus der Gruppe Laurylalkohol, Myristylalkohol und/oder Cetylalkohol ausgewählt, besonders bevorzugt sind Myristylalkohol und/oder Cetylalkohol.

In der erfindungsgemäßen Conditioner-Zubereitung sind der oder die Fettalkohol(e) aufweisend ≥ 18 Kohlenstoffatome mit einem Gesamtgehalt von 0,1 bis 1,2 Gew.-%, bevorzugt 0,3 bis 1,0 Gew.-% enthalten, bezogen auf das Gesamtgewicht der Zubereitung.

In der erfindungsgemäßen Conditioner-Zubereitung sind der oder die Fettalkohol(e) aufweisend ≤ 16 Kohlenstoffatome mit einem Gesamtgehalt von 2,0 bis 6,0 Gew.-%, bevorzugt 3,0 bis 5,5 Gew.-% enthalten, bezogen auf das Gesamtgewicht der Zubereitung.

In der erfindungsgemäßen Conditioner-Zubereitung ist der Gewichtsanteil der Fettalkohole aufweisend ≤ 16 Kohlenstoffatome größer als derjenige der Fettalkohole aufweisend ≥ 18 Kohlenstoffatome. Es ist vorteilhaft, wenn das Gewichtsverhältnis der Fettalkohole aufweisend ≤ 16 Kohlenstoffatome zu den Fettalkoholen aufweisend ≥ 18 Kohlenstoffatome von 3:1 bis 9:1 bevorzugt 5:1 bis 7:1 beträgt.

In der erfindungsgemäßen Conditioner-Zubereitung ist wenigstens ein Alkylamidoamin enthalten. Alkylamidoamine können als kationische Tenside klassifiziert werden. Alkylamidoamine weisen bei den pH-Werten, die üblicherweise in Conditioner-Zubereitungen vorliegen (pH-Werte zwischen 3,5 und 4,5) eine positive Ladung auf und zudem einen langkettigen Kohlenstoffrest. Damit weisen diese Moleküle Strukturen auf, die eine Wechselwirkung mit der Haaroberfläche begünstigen und somit können diese Moleküle einen Beitrag zur Pflegewirkung der Conditioner-Zubereitung leisten.

Bevorzugt werden Alkylamidopropyl Dimethylamine ausgewählt, die sich durch folgende allgemeine Formel darstellen lassen:

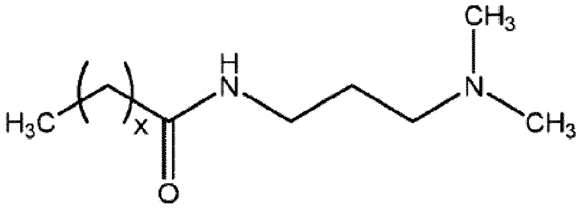

wobei x für eine ganze Zahl zwischen 14 und 22 steht. Insbesondere bevorzugt ist das Alkylamidoamin Stearamidopropyl Dimethylamin, das beispielsweise unter der Handelsbezeichnung Tego Amid S18 bei der Firma Evonok bezogen werden kann.

In der erfindungsgemäßen Conditioner-Zubereitung ist das wenigstens eine Alkylamidoamin mit einem Gesamtgehalt von 0,1 bis 5,0 Gew.-%, bevorzugt 0,3 bis 2,0 Gew.-% enthalten, bezogen auf das Gesamtgewicht der Zubereitung.

In der erfindungsgemäßen Conditioner-Zubereitung kann zusätzlich ein weiteres kationisches Tensid enthalten sein, das kein Alkylamidoamin ist. Bevorzugt wird dieses weitere kationische Tensid aus der Klasse der quartären Ammoniumverbindungen ausgewählt, die sich durch die folgende Formel darstellen lassen:

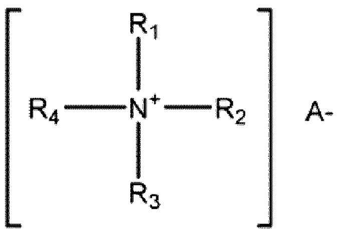

wobei die Reste $R_1$ $R_2$, $R_3$ und $R_4$ jeweils unabhängig voneinander für Wasserstoff, eine Methylgruppe oder für einen gesättigten, verzweigten oder unverzweigten Alkylrest mit einer Kettenlänge von 8 bis 30 Kohlenstoffatomen stehen, wobei mindestens einer der Reste $R_1$, $R_2$, $R_3$ und $R_4$ nicht für Wasserstoff steht, und
A- für ein physiologisch verträgliches Anion steht, beispielsweise für ein Halogenid, wie Chlorid oder Bromid sowie für Methosulfate.

Weiter bevorzugt sind drei der Reste $R_1$ bis $R_4$ Methylgruppen und ein Rest ein Alkylrest mit einer Kettenlänge von 8 bis 30 Kohlenstoffatomen. Insbesondere weiter bevorzugt wird die quartäre Ammoniumverbindung aus der Gruppe Cetrimonium Chlorid und/oder Behentrimoniun Chlorid ausgewählt.

Wenn in der erfindungsgemäßen Conditioner-Zubereitung wenigstens eine quartäre Ammoniumverbindung enthalten ist, so liegt sie mit einem Gesamtgehalt von 0,1 bis 5 Gew.-%, bevorzugt 1,0 bis 2,0 Gew.-% vor, bezogen auf das Gesamtgewicht der Zubereitung.

In der erfindungsgemäßen Zubereitung kann zusätzlich wenigstens ein Moisturizer enthalten sein. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Milchsäure und/oder Lactate, insbesondere Natriumlactat, Biosaccaride Gum-1, Glycine Soja, Ethylhexyloxyglycerin, Pyrrolidoncarbonsäure und Harnstoff. Bevorzugt wird der wenigstens eine Moisturizer aus der Gruppe Glycerin, Sorbitol, Butylenglykol, Propylenglykol, Milchsäure und/oder Salze der Milchsäure ausgewählt. Es können auch zwei oder mehr verschiedene Moisturizer enthalten sind, beispielsweise Milchsäure und Glycerin.

Wenn in der erfindungsgemäßen Conditioner-Zubereitung wenigstens ein Moisturizer enthalten ist, so liegt er

mit einem Gesamtgehalt von 0,1 bis 10 Gew. %, bevorzugt 0,5 bis 5,0 Gew. %, bezogen auf das Gesamtgewicht der Zubereitung, vor.

In der erfindungsgemäßen Zubereitung kann zusätzlich wenigstens ein Verdicker enthalten sein. Verdicker können auch als "Hydrokolloide" bezeichnet werden. Hydrokolloide sind Makromoleküle, die eine weitgehend lineare Gestalt haben und über intermolekulare und intramolekulare Wechselwirkungskräfte verfügen. Auf diese Weise kann ein netzartiges Gebilde ausgebildet werden. Hydrokolloide sind wasserlösliche oder Wasser-quellbare natürliche oder synthetische Polymere, die in wässrigen Systemen Gele oder viskose Lösungen bilden. Solche wasserlöslichen Polymere stellen eine große Gruppe chemisch sehr unterschiedlicher natürlicher und synthetischer Polymere dar, deren gemeinsames Merkmal ihre Löslichkeit oder Quellbarkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind.

Die Gruppe der kosmetisch und dermatologisch relevanten Hydrokolloide, die natürlich oder Natur-basiert sind, lässt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- anorganische Verbindungen, wie z. B. Polykieselsäuren, Tonmineralien wie Montmorillonite, Zeolithe, Kieselsäuren.

Es ist bevorzugt, wenn in der erfindungsgemäßen Conditioner-Zubereitung der wenigstens eine Verdicker aus der Gruppe der Celluloseether, wie beispielsweise Hydroxyethyl- und -propylcellulose gewählt wird.

Wenn in der erfindungsgemäßen Conditioner-Zubereitung wenigstens ein Celluloseether enthalten ist, so liegt er mit einem Gesamtgehalt von 0,1 bis 2,0 Gew.-%, bevorzugt 0,5 bis 1,0 Gew.-% vor, bezogen auf das Gesamtgewicht der Zubereitung.

In der erfindungsgemäßen Conditioner-Zubereitung kann vorteilhaft wenigstens ein Konservierungsmittel enthalten sein. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können weiter vorteilhaft Phenoxyethanol, Methyl-, Ethyl-, Propylparaben, Benzylalkohol, Sorbinsäure und/oder Salze der Sorbinsäure, beispielsweise Kalium Sorbat, Benzoesäure und/oder Salze der Benzoesäure, beispielsweise Natrium Benzoat, Salicylsäure und/oder Salze der Salicylsäure, beispielsweise Natrium Salicylat eingesetzt werden. Es ist insbesondere vorteilhaft Phenoxyethanol einzusetzen.

Wenn in der erfindungsgemäßen Conditioner-Zubereitung wenigstens ein Konservierungsmittel enthalten ist, so liegt das wenigstens eine Konservierungsmittel mit einem Gehalt von 0,01 bis 5,0 Gew. %, bevorzugt von 0,1 bis 1,0 Gew. %, besonders bevorzugt von 0,3 bis 0,7 Gew. % in der erfindungsgemäßen Zubereitung vor, bezogen auf das Gesamtgewicht der Zubereitung.

Zusätzlich kann wenigstens eine physiologisch verträgliche anorganische Säure oder organische Säure enthalten sein. Die Säuren werden benötigt, um den pH-Wert der Zubereitung auf einen sauren pH-Wert im physiologischen Bereich einzustellen. Vorteilhaft ist ein pH-Wert-Bereich von 3,5 bis 4,5. Milchsäure wirkt als Moisturizer und gleichzeitig zur Stabilisierung des pH-Wertes.

Es ist vorteilhaft, wenn Citronensäure und/oder Milchsäure ausgewählt werden, um den pH-Wert einzustellen.

Weiterhin ist es vorteilhaft, wenn die Zubereitung ein Parfüm enthält. Parfüme sind Mischungen aus Parfümrohstoffen. Das Parfüm liegt vorteilhaft mit einem Gehalt von 0,05 bis 1 Gew.-% vor, bezogen auf das Gesamtgewicht der Zubereitung.

Die erfindungsgemäße Zubereitung kann auf jede für eine derartige Zubereitung im StdT bekannte und geeignete Weise hergestellt werden.

Der erfindungsgemäßen Conditioner-Zubereitung werden keine Silikon-Verbindungen zugesetzt, ebenso sind keine Silikon-Verbindungen enthalten, die mit an sich anderen Rohstoffen in die Zubereitung gelangen. Die erfindungsgemäße Conditioner-Zubereitung ist also frei von Silikon-Verbindungen. Silikon-Verbindungen zeichnen sich durch das Vorhandensein von Silicium- und Sauerstoffatomen aus, die miteinander verknüpft sind und eine Vielzahl verschiedener Verbindungen ausbilden können.

Weiterhin werden der erfindungsgemäßen Conditioner-Zubereitung werden keine PEG-haltigen Verbindungen zugesetzt, ebenso sind keine PEG-haltigen Verbindungen enthalten, die mit an sich anderen Rohstoffen in die Zubereitung gelangen. Die erfindungsgemäße Conditioner-Zubereitung ist also frei von PEG-haltigen-Verbindungen. PEG ist die Abkürzung für Polyethylenglycol; viele der Struktureinheiten ($-CH_2-CH_2-O-$) sind dabei miteinander verknüpft. Diese Strukturen können bewirken, dass eine Penetration durch die Haut bewirkt oder verstärkt wird. Unter PEG-haltige Verbindungen werden Polyethylenglykole und Polyethylenglykolderivate verstanden.

Ebenso werden den Conditioner-Zubereitung werden keine Polyquaternium-Verbindungen zugesetzt, auch sind keine Polyquaternium-Verbindungen enthalten, die mit an sich anderen Rohstoffen in die Zubereitung gelangen. Polyquaternium-Verbindungen sind polymere Verbindungen, die sich aus einem oder verschiedenen Monomeren zusammensetzen. Ihre chemische Struktur ist verschieden; das gemeinsame Merkmal ist jedoch das Vorhandensein von Ammonium-Gruppen. Die erfindungsgemäßen Conditioner-Zubereitungen sind also frei von Polyquaternium-Verbindungen.

Eine ausgewählte Conditioner-Zubereitung (siehe Beispiel 1) wurde hergestellt und an 82 Frauen (Alter 18 bis 65 Jahre), die üblicherweise Conditioner verwenden, verteilt. Alle Frauen hatten feines oder normales Haar. Die Anwenderinnen sollten das Conditioner-Produkt über einen Zeitraum von 10 Tagen anwenden und anschließend einen Fragebogen ausfüllen. 76 Teilnehmerinnen gaben eine Rückmeldung durch einen ausgefüllten Fragebogen. Dieser Fragebogen enthielt Aussagen, die sich in erster Linie auf den Haarzustand nach Anwendung des Produktes bezogen. Die jeweiligen Aussagen sollten bestätigt (ja) oder verneint werden. Aus der Vielzahl der Fragen wurden diejenigen ausgewählt, die eine Aussage zu den Pflegeeigenschaften des Conditioners erlauben und Aussagen, die die Textur der Conditioner-Zubereitung beschreiben.

In Bezug auf die Textur gaben 84% der Anwenderinnen an, dass mit dem Conditioner eine "federleichte" Pflege für das Haar bereitsteht. Darüber hinaus wird die Verteilbarkeit des Conditioners im Haar vom 92% der Anwenderinnen als einfach zu verteilen beschrieben und die Auswaschbarkeit des Conditioners wird von 92% der Anwenderinnen als eine einfache Auswaschbarkeit beschrieben.

Hinsichtlich der Pflegeleistung bestätigten 93% der Anwenderinnen, dass der Conditioner die Haare mild pflegt und 92% sagten aus, dass der Conditioner eine ausgewogene Pflege der Haare bewirkt. Der Zustand des Haares nach der Conditioner-Anwendung wird folgendermaßen beschrieben: Mein Haar fühlt sich glatt an (97% der Anwenderinnen), meine Haar sieht gesund aus (92% der Anwenderinnen), mein Haar fühlt sich gesund an (93% der Anwenderinnen), meine Kopfhaut fühlt sich gesund an 92% der Anwenderinnen), der Conditioner bewirkt einen gesunden Glanz des Haares (91% der Anwenderinnen), der Conditioner schützt meine Haare, weil die Kämmbarkeit verbessert wird (92% der Anwenderinnen), der Conditioner pflegt die Haare insgesamt (97% der Anwenderinnen), außerdem bewirkt der Conditioner, dass die Haare schön aussehen (92% der Anwenderinnen). Diese Aussagen zeigen, dass die Anwenderinnen die pflegende Wirkung eines ausgewählten Conditioner-Produktes wahrnehmen und bestätigen.

Zusammenfassend ist die vorliegende Patentanmeldung auf den Gegenstand gerichtet, der in den folgenden, nummerierten Paragrafen beschrieben ist:

1. Kosmetische Conditioner-Zubereitung für die menschlichen Haaren, insbesondere feine und/oder strapazierte oder geschädigte Haare, enthaltend

- eine Kombination von Fettalkoholen, wobei ein oder mehrere Fettalkohol(e) aufweisend ≥ 18 Kohlenstoffatome mit einem Gesamtgehalt von weniger als 1,5 Gew.-% und ein oder mehrere Fettalkohol(e) aufweisend ≤ 16 Kohlenstoffatome mit einem Gesamtgehalt von weniger als 8,0 Gew.-% vorliegen, jeweils bezogen auf das Gesamtgewicht der Zubereitung,
- wenigstens ein Esteröl, wobei das wenigstens eine Esteröl Ester von einwertigen Alkoholen mit Fettsäuren umfasst und
- wenigstens ein Alkylamidoamin.

2. Zubereitung nach Paragraf 1 dadurch gekennzeichnet, dass der Wassergehalt von 60 bis 95 Gew.-%, insbesondere von 80 bis 90 Gew.-% beträgt, bezogen das Gesamtgewicht der Zubereitung.

3. Zubereitung nach Paragraf 1 und/oder 2 dadurch gekennzeichnet, dass die Zubereitung in Form einer Emulsion vor, insbesondere in Form einer O/W-Emulsion.

4. Zubereitung nach wenigstens einem der vorstehenden Paragrafen dadurch gekennzeichnet, dass zusätzlich wenigstens ein O/W-Emulgator, bevorzugt der Emulgator Polyglyceryl-3 Methylglucose Distearat enthalten ist.

5. Zubereitung nach wenigstens einem der vorstehenden Paragrafen dadurch gekennzeichnet, dass der wenigstens eine O/W-Emulgator mit einem Gesamtgehalt von 0,05 bis 1,0 Gew.-%, bevorzugt 0,1 bis 0,4 Gew.-% enthalten ist, bezogen auf das Gesamtgewicht der Zubereitung.

6. Zubereitung nach wenigstens einem der vorstehenden Paragrafen dadurch gekennzeichnet, dass der Alkohol in dem wenigstens einen Esteröl ein einwertiger Alkohol mit 2 bis 24 Kohlenstoffatomen, bevorzugt mit 2 bis 10 Kohlenstoffatomen, besonders bevorzugt ein verzweigter, einwertiger Alkohol mit 2 bis 10 Kohlenstoffatomen ist.

7. Zubereitung nach wenigstens einem der vorstehenden Paragrafen dadurch gekennzeichnet, dass die Fettsäure in dem wenigstens einen Esteröl eine Fettsäure mit 6 bis 30 Kohlenstoffatomen, bevorzugt mit 6 bis 18 Kohlenstoffatomen, besonders bevorzugt eine unverzweigte und gesättigte Fettsäure mit 6 bis 18 Kohlenstoffatomen ist.

8. Zubereitung nach wenigstens einem der vorstehenden Paragrafen dadurch gekennzeichnet, dass das wenigstens eine Esteröl ausgewählt wird aus der Gruppe, die Isopropylmyristat, Isopropylpalmitat, Isopropylstearat, 2-Ethylhexylpalmitat, 2-Ethylhexyllaurat, 2-Ethylhexylstearat, und/oder Isoamyllaurat umfasst, insbesondere aus Isopropylmyristat, Isopropylpalmitat, Isopropylstearat, 2-Ethylhexylpalmitat, 2-Ethylhexyllaurat, 2-Ethylhexylstearat, und/oder Isoamyllaurat besteht, am meisten bevorzugt ist das wenigstens eine Esteröl Isoamyllaurat, Isopropylmyristat und/oder Isopropylpalmitat.

9. Zubereitung nach wenigstens einem der vorstehenden Paragrafen dadurch gekennzeichnet, dass das wenigstens eine Esteröl mit einem Gesamtgehalt von 0,1 bis 2,0 Gew.-%, bevorzugt 0,5 bis 1,0 Gew.-% enthalten ist, bezogen auf das Gesamtgewicht der Zubereitung.

10. Zubereitung nach wenigstens einem der vorstehenden Paragrafen dadurch gekennzeichnet, dass die Fettalkohole unverzweigt oder verzweigt und/oder gesättigt oder ungesättigt sind.

11. Zubereitung nach wenigstens einem der vorstehenden Paragrafen dadurch gekennzeichnet, dass der oder die Fettalkohol(e) aufweisend ≥ 18 Kohlenstoffatome aus der Gruppe Stearylalkohol, Arachidylalhohol und/oder Behenylalkohol ausgewählt wird/werden, bevorzugt ist Stearylalkohol.

12. Zubereitung nach wenigstens einem der vorstehenden Paragrafen dadurch gekennzeichnet, dass der oder die Fettalkohol(e) aufweisend ≤ 16 Kohlenstoffatome aus der Gruppe Laurylalkohol, Myristylalkohol und/oder Cetylalkohol ausgewählt wird/werden, bevorzugt sind Myristylalkohol und/oder Cetylalkohol.

13. Zubereitung nach wenigstens einem der vorstehenden Paragrafen dadurch gekennzeichnet, dass der oder die Fettalkohol(e) aufweisend ≥ 18 Kohlenstoffatome mit einem Gesamtgehalt von 0,1 bis 1,2 Gew.-%, bevorzugt 0,3 bis 1,0 Gew.-% enthalten ist/sind, bezogen auf das Gesamtgewicht der Zubereitung.

14. Zubereitung nach wenigstens einem der vorstehenden Paragrafen dadurch gekennzeichnet, dass der oder die Fettalkohol(e) aufweisend ≤ 16 Kohlenstoffatome mit einem Gesamtgehalt von 2,0 bis 6,0 Gew.-%, bevorzugt 3,0 bis 5,5 Gew.-% enthalten ist/sind, bezogen auf das Gesamtgewicht der Zubereitung.

15. Zubereitung nach wenigstens einem der vorstehenden Paragrafen dadurch gekennzeichnet, dass der Gewichtsanteil der Fettalkohole aufweisend ≤ 16 Kohlenstoffatome größer als der Gewichtsanteil der Fettalkohole aufweisend ≥ 18 Kohlenstoffatome ist, bevorzugt ist das Gewichtsverhältnis der Fettalkohole aufweisend ≤ 16 Kohlenstoffatome zu den Fettalkoholen aufweisend ≥ 18 Kohlenstoffatome von 3:1 bis 9:1, insbesondere 5:1 bis 7:1.

16. Zubereitung nach wenigstens einem der vorstehenden Paragrafen dadurch gekennzeichnet, dass das wenigstens eine Alkylamidoamin mit einem Gesamtgehalt von 0,1 bis 5,0 Gew.-%, bevorzugt 0,3 bis 2,0 Gew.-% enthalten ist, bezogen auf das Gesamtgewicht der Zubereitung.

17. Zubereitung nach wenigstens einem der vorstehenden Paragrafen dadurch gekennzeichnet, dass das Alkylamidoamin Stearamidopropyl Dimethylamin ist.

18. Zubereitung nach wenigstens einem der vorstehenden Paragrafen dadurch gekennzeichnet, dass zusätzlich wenigstens ein weiteres kationisches Tensid enthalten ist, das kein Alkylamidoamin ist, bevorzugt wird das wenigstens eine weitere kationische Tensid aus der Gruppe der quartären Ammoniumverbindungen ausgewählt, weiter bevorzugt aus der Gruppe Cetrimonium Chlorid und/oder Behentrimoniun Chlorid.

19. Zubereitung nach wenigstens einem der vorstehenden Paragrafen dadurch gekennzeichnet, dass die wenigstens eine quartäre Ammoniumverbindung mit einem Gesamtgehalt von 0,1 bis 5 Gew.-%, bevorzugt 1,0 bis 2,0 Gew.-% vorliegt, bezogen auf das Gesamtgewicht der Zubereitung.

20. Zubereitung nach wenigstens einem der vorstehenden Paragrafen dadurch gekennzeichnet, dass zusätzlich wenigstens ein Moisturizer, bevorzugt ausgewählt aus Glycerin, Sorbitol, Butylenglykol, Propylenglykol, Milchsäure und/oder Lactate, Biosaccaride Gum-1, Glycine Soja, Ethylhexyloxyglycerin und/oder Harnstoff, weiter bevorzugt

aus der Gruppe Glycerin, Sorbitol, Butylenglykol, Propylenglykol, Milchsäure und/oder Salze der Milchsäure, enthalten ist.

21. Zubereitung nach wenigstens einem der vorstehenden Paragrafen dadurch gekennzeichnet, dass der wenigstens eine Moisturizer mit einem Gesamtgehalt von 0,1 bis 10 Gew. %, bevorzugt 0,5 bis 5,0 Gew. % enthalten ist, bezogen auf das Gesamtgewicht der Zubereitung.

22. Zubereitung nach wenigstens einem der vorstehenden Paragrafen dadurch gekennzeichnet, dass zusätzlich wenigstens ein Verdicker, bevorzugt ausgewählt aus der Gruppe der Celluloseether enthalten ist.

23. Zubereitung nach wenigstens einem der vorstehenden Paragrafen dadurch gekennzeichnet, dass der wenigstens eine Verdicker mit einem Gesamtgehalt von 0,1 bis 2,0 Gew.-%, bevorzugt 0,5 bis 1,0 Gew.-% vorliegt, bezogen auf das Gesamtgewicht der Zubereitung.

24. Zubereitung nach wenigstens einem der vorstehenden Paragrafen dadurch gekennzeichnet, dass die Zubereitung frei von Silikon-Verbindungen ist.

25. Zubereitung nach wenigstens einem der vorstehenden Paragrafen dadurch gekennzeichnet, dass die Zubereitung frei von PEG-haltigen-Verbindungen ist.

26. Zubereitung nach wenigstens einem der vorstehenden Paragrafen dadurch gekennzeichnet, dass die Zubereitung frei vom Polyquaternium-Verbindungen ist.

**Beispiele:**

Die untenstehenden Beispiele sollen die Erfindung illustrieren und nicht einschränken. Die Angaben beziehen sich auf Gew.-% und den jeweiligen Aktivgehalt, soweit nicht anders angegeben.

| INCI | Example 1 | Example 2 |
|---|---|---|
| Cetyl Alcohol | 3,3 | 2,9 |
| Myristyl Alcohol | 0,7 | 0,5 |
| Stearyl Alcohol | 0,7 | 0,5 |
| Isoamyl Laurate | 0,6 | 0,4 |
| Isopropyl Myristate | 0,7 | 0,5 |
| Stearamidopropyl Dimethylamine | 0,4 | 0,8 |
| Lactic Acid | 0,18 | 0,27 |
| Polyglyceryl-3 Methylglucose Distearate | 0,2 | 0,2 |
| Behentrimonium Chloride | 2 | 1,5 |
| Hydroxyethylcellulose | 0,6 | 0,5 |
| Glycerin | | 1,5 |
| Citric Acid | 0,3 | 0,1 |
| Sodium Citrate | 0,35 | |
| Panthenol | 0,2 | |
| Phenoxyethanol | 0,5 | 0,5 |
| Parfum | 0,5 | 0,8 |
| Aqua | ad 100 | ad 100 |

**Patentansprüche**

1. Kosmetische Conditioner-Zubereitung für die menschlichen Haaren, insbesondere feine und/oder strapazierte oder geschädigte Haare, enthaltend

   - eine Kombination von Fettalkoholen, wobei ein oder mehrere Fettalkohol(e) aufweisend ≥ 18 Kohlenstoffatome mit einem Gesamtgehalt von weniger als 1,5 Gew.-% und ein oder mehrere Fettalkohol(e) aufweisend ≤ 16 Kohlenstoffatome mit einem Gesamtgehalt von weniger als 8,0 Gew.-% vorliegen, jeweils bezogen auf das Gesamtgewicht der Zubereitung,
   - wenigstens ein Esteröl, wobei das wenigstens eine Esteröl Ester von einwertigen Alkoholen mit Fettsäuren umfasst und
   - wenigstens ein Alkylamidoamin.

2. Zubereitung nach Anspruch 1, **dadurch gekennzeichnet, dass** der Alkohol in dem wenigstens einen Esteröl ein einwertiger Alkohol mit 2 bis 24 Kohlenstoffatomen, bevorzugt mit 2 bis 10 Kohlenstoffatomen, besonders bevorzugt ein einwertiger, verzweigter Alkohol mit 2 bis 10 Kohlenstoffatomen ist.

3. Zubereitung nach Anspruch 1 und/oder 2, **dadurch gekennzeichnet, dass** die Fettsäure in dem wenigstens einen Esteröl eine Fettsäure mit 6 bis 30 Kohlenstoffatomen, bevorzugt mit 6 bis 18 Kohlenstoffatomen, besonders bevorzugt eine unverzweigte und gesättigte Fettsäure mit 6 bis 18 Kohlenstoffatomen ist.

4. Zubereitung nach wenigstens einem der vorstehenden Ansprüche, **dadurch gekennzeichnet, dass** das wenigstens eine Esteröl ausgewählt wird aus der Gruppe, die Isopropylmyristat, Isopropylpalmitat, Isopropylstearat, 2-Ethylhexylpalmitat, 2-Ethylhexyllaurat, 2-Ethylhexylstearat, und/oder Isoamyllaurat umfasst, insbesondere aus Isopropylmyristat, Isopropylpalmitat, Isopropylstearat, 2-Ethylhexylpalmitat, 2-Ethylhexyllaurat, 2-Ethylhexylstearat, und/oder Isoamyllaurat besteht, am meisten bevorzugt ist das wenigstens eine Esteröl Isoamyllaurat, Isopropylmyristat und/oder Isopropylpalmitat.

5. Zubereitung nach wenigstens einem der vorstehenden Ansprüche, **dadurch gekennzeichnet, dass** der oder die Fettalkohol(e) aufweisend ≥ 18 Kohlenstoffatome aus der Gruppe Stearylalkohol, Arachidylalhohol und/oder Behenylalkohol ausgewählt wird/werden, bevorzugt ist Stearylalkohol.

6. Zubereitung nach wenigstens einem der vorstehenden Ansprüche, **dadurch gekennzeichnet, dass** der oder die Fettalkohol(e) aufweisend ≤ 16 Kohlenstoffatome aus der Gruppe Laurylalkohol, Myristylalkohol und/oder Cetylalkohol ausgewählt wird/werden, bevorzugt sind Myristylalkohol und/oder Cetylalkohol.

7. Zubereitung nach wenigstens einem der vorstehenden Ansprüche, **dadurch gekennzeichnet, dass** das wenigstens eine Alkylamidoamin mit einem Gesamtgehalt von 0,1 bis 5,0 Gew.-%, bevorzugt 0,3 bis 2,0 Gew.-% enthalten ist, bezogen auf das Gesamtgewicht der Zubereitung.

8. Zubereitung nach wenigstens einem der vorstehenden Ansprüche, **dadurch gekennzeichnet, dass** das Alkylamidoamin Stearamidopropyl Dimethylamin ist.

9. Zubereitung nach wenigstens einem der vorstehenden Ansprüche, **dadurch gekennzeichnet, dass** die Zubereitung frei von Silikon-Verbindungen ist.

10. Zubereitung nach wenigstens einem der vorstehenden Ansprüche, **dadurch gekennzeichnet, dass** die Zubereitung frei von PEG-haltigen-Verbindungen ist.

11. Zubereitung nach wenigstens einem der vorstehenden Ansprüche, **dadurch gekennzeichnet, dass** die Zubereitung frei von Polyquaternium-Verbindungen ist.

Europäisches Patentamt
European Patent Office
Office européen des brevets

# EUROPÄISCHER RECHERCHENBERICHT

Nummer der Anmeldung
EP 22 16 7866

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 199 55 842 A1 (KAO CORP [JP]) 7. Juni 2001 (2001-06-07) | 1-11 | INV. A61Q5/12 A61K8/34 A61K8/37 A61K8/42 |
| Y | * Absatz [0043]; Beispiel 1 * | 1-11 | |
| X | DE 200 17 586 U1 (GOLDWELL GMBH [DE]) 28. Februar 2002 (2002-02-28) * Absätze [0100] - [0113] * | 1-11 | |
| X | DE 203 01 831 U1 (CCL RAPID SPRAY GMBH & CO KG [DE]) 17. April 2003 (2003-04-17) | 1-8,10, 11 | |
| Y | * Seite 5 - Seite 6; Beispiel 2 * | 1-11 | |

RECHERCHIERTE SACHGEBIETE (IPC)

A61Q
A61K

Der vorliegende Recherchenbericht wurde für alle Patentansprüche erstellt

1

| Recherchenort | Abschlußdatum der Recherche | Prüfer |
|---|---|---|
| München | 17. Oktober 2022 | Bader, Karl Günther |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 22 16 7866

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-10-2022

| Im Recherchenbericht angeführtes Patentdokument | Datum der Veröffentlichung | Mitglied(er) der Patentfamilie | Datum der Veröffentlichung |
|---|---|---|---|
| DE 19955842 A1 | 07-06-2001 | KEINE | |
| DE 20017586 U1 | 28-02-2002 | KEINE | |
| DE 20301831 U1 | 17-04-2003 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2138156 A1
- EP 0786250 A1
- DE 102017212403 A1
- CN 2019121114 W
- CN 2019121115 W